# EUROPEAN PATENT SPECIFICATION

(11) **EP 2 979 659 B1**
(45) Date of publication and mention of the grant of the patent: **27.05.2020**
(21) Application number: 15179277.7
(22) Date of filing: 31.07.2015
(51) Int. Cl.: A61F 7/00

(54) **ELEKTROSURGICAL GENERATOR FOR IMPROVING HIGH FREQUENCY LEAKAGE**
ELEKTROCHIRURGISCHE GENERATOREN ZUR VERBESSERUNG VON HOCHFREQUENTER LECKAGE
GÉNÉRATEURS ÉLECTROCHIRURGICAUX D'AMÉLIORATION DES FUITES HAUTE FRÉQUENCE

(30) Priority: 01.08.2014 US 201462031939 P; 11.06.2015 US 201514736417
(43) Date of publication of application: 03.02.2016
(73) Proprietor: Covidien LP, Mansfield, MA 02048 (US)
(72) Inventor: Gilbert, James A., Boulder, CO 80305 (US); Johnson, Joshua H., Arvada, CO 80005 (US)
(74) Representative: Morgan, Marc

(56) References cited:
- EP-A1- 1 064 047
- EP-A1- 2 742 888
- WO-A1-98/15317
- US-A- 5 841 239
- US-A1- 2007 129 716
- US-A1- 2007 173 808
- US-A1- 2010 124 035
- US-A1- 2013 023 871
- US-A1- 2013 267 945

## Description

### BACKGROUND

### 1. Technical Field

The present disclosure relates to radiofrequency amplifiers that use phase-shifted full bridge resonant inverters. Particularly, the present disclosure is directed to reducing common mode currents and high frequency leakage in radiofrequency amplifiers.

### 2. Background of the Related Art

Energy-based tissue treatment is well known in the art. Various types of energy (e.g., electrical, ultrasonic, microwave, cryogenic, thermal, laser, etc.) are applied to tissue to achieve a desired result. Electrosurgery involves application of high radio frequency electrical current to a surgical site to cut, ablate, coagulate or seal tissue. A source or active electrode delivers radio frequency energy from the electro surgical generator to the tissue and a return electrode carries the current back to the generator. In monopolar electrosurgery, the source electrode is typically part of the surgical instrument held by the surgeon and applied to the tissue to be treated and the return electrode is placed remotely from the active electrode to carry the current back to the generator. In bipolar electrosurgery, one of the electrodes of the hand-held instrument functions as the active electrode and the other as the return electrode.

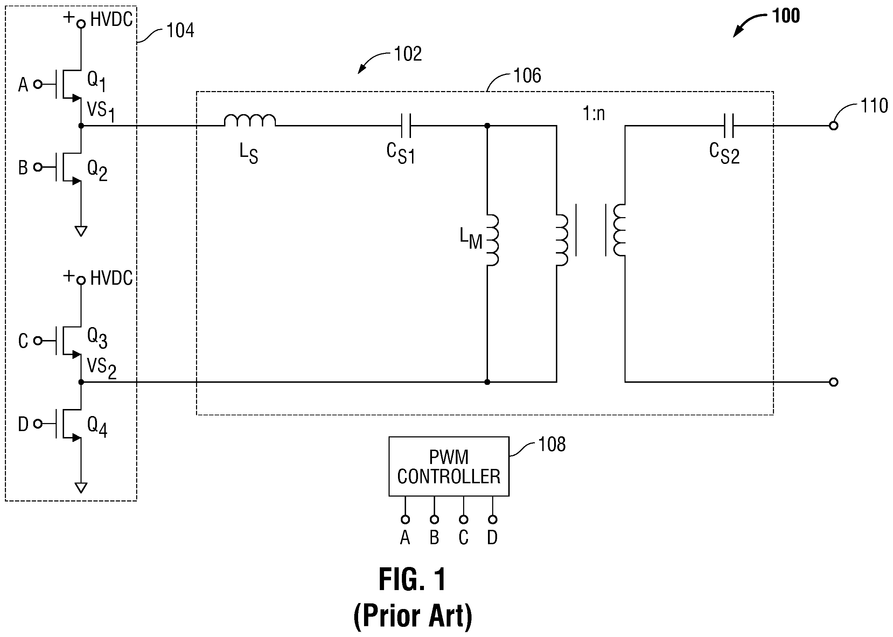

Fig. 1 is an example of a prior art electrosurgical generator that uses a phase-shifted full bridge resonant inverter to generate the electrosurgical energy needed to perform the electrosurgical procedure. The generator 100 includes a resonant inverter circuit 102 and a pulse width modulation (PWM) controller 108. The resonant inverter circuit 102 includes an H-bridge 104 and a tank 106. In tank 106, a series capacitor C_{S2} is placed in series with the secondary of the transformer T₁ to prevent DC current from flowing into the output of the active terminal 110 and possibly harming a patient or an end user. However, the presence of capacitor C_{S2} introduces a slight imbalance in the charge and high frequency output that may contribute to the potential flow of common mode currents and high frequency leakage current imbalances while under load. US2014107641 A1, US2007129716 A1, US2013267945 A1 and US2007173808 A1 refer to prior art documents relevant for the present invention.

### SUMMARY

The present invention is defined by independent claim 1. Preferred embodiments are disclosed in the dependent claims. In an aspect of the present disclosure, an electrosurgical generator includes an H-bridge and a tank driven by the H-bridge. The tank includes a transformer having a primary winding and a secondary winding which includes a first coil and a second coil. The tank also includes a capacitor connected in series between the first coil and the second coil.

In the aspects described herein, a controller is configured to drive the H-bridge.

In some aspects, the electrosurgical generator also includes a charge drain electrically coupled to the capacitor. The charge drain includes a switching member which may be any conventional switch or a relay. A controller may control the switching member wherein the controller closes the switching member when the electrosurgical generator is inactive and opens the switching member when the electrosurgical generator is active. The charge drain may also include a resistor or a positive temperature coefficient thermistor or resettable fuse.

In some aspects, the generator also includes a printed circuit board. The printed circuit board includes a plurality of contacts configured to receive a secondary winding of a transformer. The plurality of contacts include: a first contact configured to be electrically coupled to an active terminal; a second contact configured to be electrically coupled to a return terminal; a third contact; and a fourth contact. The printed circuit board also includes a capacitor integrated into the printed circuit board. The capacitor is electrically coupled to the third contact and the fourth contact.

The third contact and the fourth contact are configured to be electrically coupled to a center tap of the transformer.

### BRIEF DESCRIPTION OF THE DRAWINGS

The above and other aspects, features, and advantages of the present disclosure will become more apparent in light of the following detailed description when taken in conjunction with the accompanying drawings in which:
Fig. 1 is a schematic illustration of a prior art electrosurgical generator;
Fig. 2 is a schematic illustration of an electrosurgical generator in accordance with an embodiment of the present disclosure;
Figs. 3A-3C are illustrations of a drain that may be used in conjunction with the electrosurgical generator of Fig. 2;
Fig. 4 is an illustration of a portion of a printed circuit board (PCB) in accordance with an embodiment of the present disclosure; and
Fig. 5 is a cross-section of the PCB of Figure 4 taken along the line 5-5.

### DETAILED DESCRIPTION

Particular embodiments of the present disclosure are described hereinbelow with reference to the accompanying drawings; however, it is to be understood that the disclosed embodiments are merely examples of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure. Like reference numerals may refer to similar or identical elements throughout the description of the figures.

This description may use the phrases "in an embodiment," "in embodiments," "in some embodiments," or "in other embodiments," which may each refer to one or more of the same or different embodiments in accordance with the present disclosure. For the purposes of this description, a phrase in the form "A/B" means A or B. For the purposes of the description, a phrase in the form "A and/or B" means "(A), (B), or (A and B)". For the purposes of this description, a phrase in the form "at least one of A, B, or C" means "(A), (B), (C), (A and B), (A and C), (B and C), or (A, B and C)".

As used herein, the term "generator" may refer to a device capable of providing energy. Such device may include a power source and an electrical circuit capable of modifying the energy outputted by the power source to output energy having a desired intensity, frequency, and/or waveform.

The systems described herein may also utilize one or more controllers to receive various information and transform the received information to generate an output. The controller may include any type of computing device, computational circuit, or any type of processor or processing circuit capable of executing a series of instructions that are stored in a memory. The controller may include multiple processors and/or multicore central processing units (CPUs) and may include any type of processor, such as a microprocessor, digital signal processor, microcontroller, or the like. The controller may also include a memory to store data and/or algorithms to perform a series of instructions.

Any of the herein described data and/or algorithms may be contained on one or more machine-readable media or memory. The term "memory" may include a mechanism that provides (e.g., stores and/or transmits) information in a form readable by a machine such as a processor, computer, or a digital processing device. For example, a memory may include a read only memory (ROM), random access memory (RAM), magnetic disk storage media, optical storage media, flash memory devices, or any other volatile or non-volatile memory storage device. Code or instructions contained thereon can be represented by carrier wave signals, infrared signals, digital signals, and by other like signals.

The present disclosure is directed to an electrosurgical generator that employs a phase-shifted full bridge resonant inverter having a tank topology and an H-bridge. In an embodiment of the present disclosure, a capacitor is placed at a center tap on the secondary side of a transformer in the tank topology. The capacitor may be a discrete component or it may be integrated into a PCB.

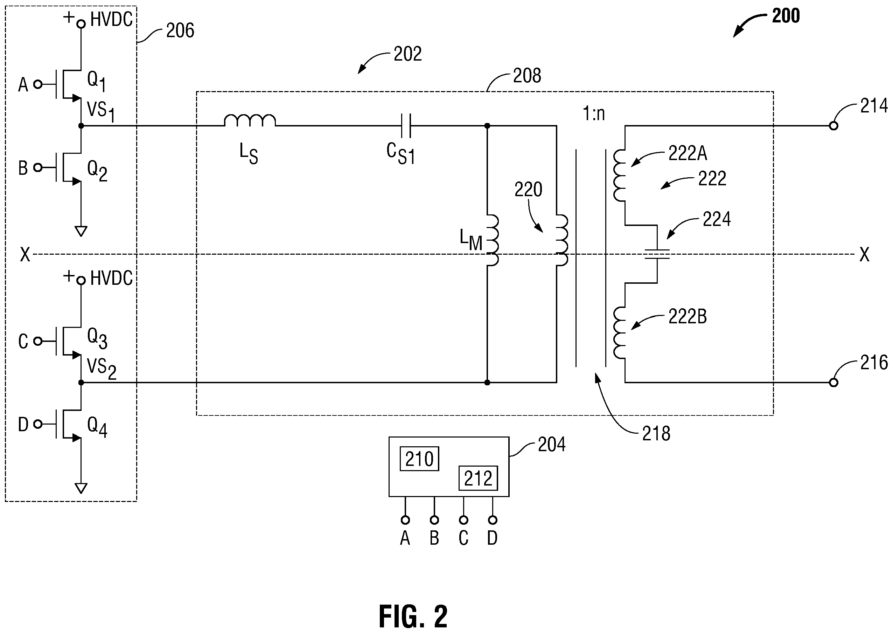

Turning to Fig. 2, an electrosurgical generator in accordance with an embodiment of the present disclosure is shown generally as 200. The generator 200 includes suitable input controls (e.g., buttons, activators, switches, touch screen, etc.) for controlling the generator 200. In addition, the generator 200 may include one or more display screens (not shown) for providing the user with variety of output information (e.g., intensity settings, treatment complete indicators, etc.). The controls allow the user to adjust power of the RF energy, waveform, as well as the level of maximum arc energy allowed which varies depending on desired tissue effects and other parameters to achieve the desired waveform suitable for a particular task, e.g., coagulating, tissue sealing, intensity setting, etc. An instrument (not shown) that may be connected to the generator 200 may also include a plurality of input controls that may be redundant with certain input controls of the generator 200. Placing the input controls at the instrument allows for easier and faster modification of RF energy parameters during the surgical procedure without requiring interaction with the generator 200.

The generator 200 may include a plurality of connectors to accommodate various types of electrosurgical instruments. Further, the generator 200 may operate in monopolar or bipolar modes by including a switching mechanism (e.g., relays) to switch the supply of RF energy between the connectors.

The generator 200 includes a resonant inverter circuit 202 and a pulse width modulation (PWM) controller 204. The resonant inverter circuit 202 includes an H-bridge 206 having FETs Q1, Q2, Q3, and Q4 and a tank 208. The PWM controller 204 includes a processor 210 and a memory 212.

In the resonant inverter circuit 202, the H-bridge 206 is supplied with a positive high voltage direct current (+HVDC). The tank 208 is driven in a full-bridge configuration by the active FET switches Q1, Q2, Q3 and Q4. The PWM controller 208 supplies phase-shifted PWM timing signals to FET switches Q1, Q2, Q3 and Q4 as shown in Fig. 2. FETs Q1 and Q2 provide a voltage V_{S1} to the tank 208 and FETs Q3 and Q4 provide a voltage V_{S2} to the tank 208.

The tank 208 outputs electrosurgical energy to an instrument (not shown) via active terminal 214. In particular, the active terminal 214 provides either continuous or pulsed sinusoidal waveforms of high RF energy. The active terminal 214 is configured to provide a plurality of waveforms having various duty cycles, peak voltages, crest factors, and other suitable parameters. Certain types of waveforms are suitable for specific electrosurgical modes. For instance, the active terminal 214 may provide a 100% duty cycle sinusoidal waveform in cut mode, which is best suited for ablating, fusing and dissecting tissue and a 1-25% duty cycle waveform in coagulation mode, which is best used for cauterizing tissue to stop bleeding.

A return terminal 216 is coupled to a return pad (not shown) for monopolar procedures. Alternatively, the return terminal 216 is electrically coupled to a return electrode (not shown) on an instrument.

The tank 208 includes a transformer 218 that is used to provide the desired voltage output as well as isolate the patient from the generator. The transformer 218 includes a primary winding 220 and a secondary winding 222. The secondary winding 222 is a split output winding that includes a first coil 222A and a second coil 222B. A capacitor 224 is placed at a center tap of the transformer 218. Specifically, the capacitor 224 is placed in series between the first coil 222A and the second coil 222B. As shown in Fig. 2, the capacitor 224 is placed across the common mode plane (represented by line X-X). By placing the capacitor 224 in series between the first and second coils 222A, 222B across the common mode plane, a current or charge imbalance between terminals 214 and 216 is mitigated thus reducing any common mode currents and high frequency leakage. Also, the presence of the capacitor 224 prevents DC current from flowing into the output of the active terminal 214 and possibly harming a patient or an end user.

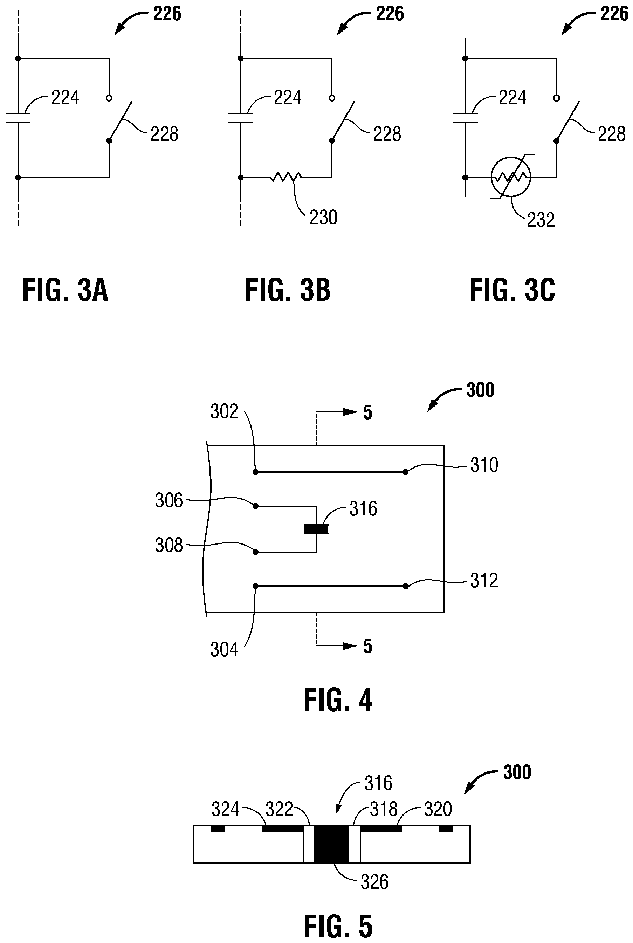

In some embodiments, a charge drain 226 is placed in parallel with the capacitor 224 to prevent the build-up of charge on the capacitor 224. (See Fig. 3A.) The charge drain 226 includes a switch 228. The switch 228 may be any conventional switch or relay. The switch 228 is normally closed when the generator 200 is inactive and open when the generator 200 is active. The switch 228 may be controlled by a PWM controller 204, another controller (not shown), or a sensor (not shown) that detects whether the generator 200 is active or inactive. Fig. 3B includes a charge drain resistor 230 to assist in the discharge of capacitor 224. Fig. 3C substitutes the charge drain resistor 230 of Fig. 3B with a positive temperature coefficient (PTC) thermistor or resettable fuse 232.

In some embodiments described above, the capacitor 224 is a discrete component. In some embodiments, the capacitor may be integrated into a PCB to reduce the number of components needed for the electrosurgical generator. In addition, in some embodiments, this will enable the capacitor to be co-located in the PCB at each of the transformer leads providing a better performance and easier matching. Figs. 4 and 5 depict a portion of a PCB 300 for an electrosurgical generator that integrates a capacitor therein. As shown in Fig. 4, the PCB 300 includes contacts 302, 304, 306, and 308 for the secondary of a transformer. Contact 302 is electrically coupled to a contact 310 which is coupled to an active terminal (not shown). Contact 304 is electrically coupled to a contact 312 which is coupled to a return terminal (not shown). Contacts 306 and 308 are configured to receive a center tap of a transformer (not shown). Contacts 306 and 308 have a capacitor 316 disposed therebetween. As shown in Fig. 5, capacitor 316 has a first plate 318 that is electrically coupled to contact 306 via trace 320. A second plate 322 is electrically coupled to contact 308 via trace 324. A dielectric material 326 is disposed between the first plate 318 and second plate 322. The dielectric material 326 may be any conventional dielectric material.

It should be understood that the foregoing description is only illustrative of the present disclosure. Various alternatives and modifications can be devised by those skilled in the art without departing from the scope of the appended claims. Accordingly, the present disclosure is intended to embrace all such alternatives, modifications and variances. The embodiments described with reference to the attached drawing figures are presented only to demonstrate certain examples of the disclosure.

## Claims

1. An electrosurgical generator (200) comprising:
an H-bridge (206); and
a tank (208) driven by the H-bridge, the tank including:
a transformer (218) having a primary winding (220) and a secondary winding (222), the secondary winding including a first coil (222A) and a second coil (222B); **characterised in that** the tank further includes
a capacitor (224) connected in series between the first coil and the second coil, wherein the capacitor is placed across a common mode plane (X-X) for mitigating a current or charge imbalance between active and return terminals (214,216) of the electrosurgical generator, thus reducing any common mode currents and high frequency leakage.

2. The electrosurgical generator of claim 1, further comprising a controller (204) configured to drive the H-bridge.

3. The electrosurgical generator of claim 1 or 2, further comprising a charge drain (226) electrically coupled to the capacitor.

4. The electrosurgical generator of claim 3, wherein the charge drain includes a switching member (228).

5. The electrosurgical generator of claim 4, wherein the switching member is a relay.

6. The electrosurgical generator of any one of claims 4 or 5 further comprising a controller configured to control the switching member wherein the controller is configured to close the switching member when the electrosurgical generator is inactive and open the switching member when the electrosurgical generator is active.

7. The electrosurgical generator of any one of claims 4, 5 or 6, wherein the charge drain includes a resistor.

8. The electrosurgical generator of any one of claims 4 to 7, wherein the charge drain includes at least one of a positive temperature coefficient thermistor or a resettable fuse.

9. The electrosurgical generator of claim 4, 5 or 6, wherein the switching member is placed in parallel with the capacitor.

10. The electrosurgical generator of claim 1, wherein a capacitor is placed at a center tap on the secondary winding of the transformer.

11. The electrosurgical generator of any preceding claim wherein the capacitor is integrated into a printed circuit board (300).

12. The electrosurgical generator of claim 1, comprising a printed circuit board (300) comprising:
a plurality of contacts (302, 304, 306, 308) receiving the secondary winding of the transformer, wherein the plurality of contacts include: a first contact (302) electrically coupled to an active terminal (214) of the electrosurgical generator; a second contact (304) electrically coupled to a return terminal (216) of the electrosurgical generator; a third contact (306); and a fourth contact (308); and
the capacitor integrated into the printed circuit board, the capacitor being electrically coupled to the third contact and the fourth contact.

13. The printed circuit board of claim 12, wherein the third contact is electrically coupled to the first coil of the transformer.

14. The printed circuit board of claim 12 or 13, wherein the fourth contact is electrically coupled to the second coil of the transformer.

## Patentansprüche

1. Elektrochirurgischer Generator (200), umfassend:
eine H-Brücke (206); und
einen Tank (208), der von der H-Brücke angetrieben wird, wobei der Tank Folgendes umfasst:
einen Transformator (218) mit einer Primärwicklung (220) und einer Sekundärwicklung (222), wobei die Sekundärwicklung eine erste Spule (222A) und eine zweite Spule (222B) umfasst;
**dadurch gekennzeichnet, dass** der Tank ferner einen Kondensator (224) umfasst, der in Reihe zwischen der ersten Spule und der zweiten Spule geschaltet ist, wobei der Kondensator quer zu einer Gleichtakt-Ebene (X-X) angeordnet ist, um ein Strom- oder Ladungsungleichgewicht zwischen Aktiv- und Rückleitungsanschlüssen (214, 216) des elektrochirurgischen Generators abzuschwächen und so jegliche Gleichtakt-Ströme und Hochfrequenz-Leckagen zu reduzieren.

2. Elektrochirurgischer Generator nach Anspruch 1, ferner umfassend eine Steuerung (204), die zum Ansteuern der H-Brücke konfiguriert ist.

3. Elektrochirurgischer Generator nach Anspruch 1 oder 2, ferner umfassend einen elektrisch an den Kondensator gekoppelten Ladungsabfluss (226).

4. Elektrochirurgischer Generator nach Anspruch 3, wobei der Ladungsabfluss ein Schaltelement (228) enthält.

5. Elektrochirurgischer Generator nach Anspruch 4, wobei das Schaltelement ein Relais ist.

6. Elektrochirurgischer Generator nach einem der Ansprüche 4 oder 5, ferner umfassend eine Steuerung, die so konfiguriert ist, dass sie das Schaltelement steuert, wobei die Steuerung so konfiguriert ist, dass sie das Schaltelement schließt, wenn der elektrochirurgische Generator inaktiv ist, und das Schaltelement öffnet, wenn der elektrochirurgische Generator aktiv ist.

7. Elektrochirurgischer Generator nach einem der Ansprüche 4, 5 oder 6, bei dem der Ladungsabfluss einen Widerstand enthält.

8. Elektrochirurgischer Generator nach einem der Ansprüche 4 bis 7, bei dem der Ladungsabfluss mindestens einen Thermistor mit positivem Temperaturkoeffizienten oder eine rückstellbare Sicherung enthält.

9. Elektrochirurgischer Generator nach Anspruch 4, 5 oder 6, bei dem das Schaltelement parallel zum Kondensator angeordnet ist.

10. Elektrochirurgischer Generator nach Anspruch 1, wobei ein Kondensator an einem Mittelabgriff der Sekundärwicklung des Transformators angeordnet ist.

11. Elektrochirurgischer Generator nach einem der vorhergehenden Ansprüche, wobei der Kondensator in eine Leiterplatte integriert ist (300).

12. Elektrochirurgischer Generator nach Anspruch 1 mit einer Leiterplatte (300), umfassend:
mehrere Kontakte (302, 304, 306, 308), die die Sekundärwicklung des Transformators aufnehmen, wobei die mehreren Kontakte Folgendes umfassen:
einen ersten Kontakt (302), der elektrisch mit einem aktiven Anschluss (214) des elektrochirurgischen Generators gekoppelt ist;
einen zweiten Kontakt (304), der elektrisch an einen Rückleitungsanschluss (216) des elektrochirurgischen Generators gekoppelt ist;
einen dritten Kontakt (306); und
einen vierten Kontakt (308); und
den in die Leiterplatte integrierten Kondensator, wobei der Kondensator mit dem dritten und vierten Kontakt elektrisch gekoppelt ist.

13. Leiterplatte nach Anspruch 12, wobei der dritte Kontakt mit der ersten Spule des Transformators elektrisch gekoppelt ist.

14. Leiterplatte nach Anspruch 12 oder 13, wobei der vierte Kontakt mit der zweiten Spule des Transformators elektrisch gekoppelt ist.

## Revendications

1. Générateur électrochirurgical (200) comprenant :
un pont en H (206) ; et
un réservoir (208) entraîné par le pont en H, le réservoir comprenant :
un transformateur (218) doté d'un enroulement primaire (220) et d'un enroulement secondaire (222), l'enroulement secondaire comportant une première bobine (222A) et une seconde bobine (222B) ;
**caractérisé en ce que** le réservoir comporte en outre
un condensateur (224) couplé en série entre la première bobine et la seconde bobine, le condensateur étant placé sur un plan de mode commun (X-X) afin d'atténuer un déséquilibre de courant ou de charge entre les bornes active et de retour (214, 216) du générateur électrochirurgical, ce qui réduit les courants de mode commun et la fuite à haute fréquence.

2. Générateur électrochirurgical selon la revendication 1, comprenant en outre un dispositif de commande (204) configuré pour activer le pont en H.

3. Générateur électrochirurgical selon la revendication 1 ou 2, comprenant en outre un drain de charge (226) couplé électriquement au condensateur.

4. Générateur électrochirurgical selon la revendication 3, dans lequel le drain de charge comporte un élément de commutation (228).

5. Générateur électrochirurgical selon la revendication 4, dans lequel l'élément de commutation est un relais.

6. Générateur électrochirurgical selon l'une quelconque des revendications 4 ou 5, comprenant en outre un dispositif de commande configuré pour commander l'élément de commutation, le dispositif de commande étant configuré pour fermer l'élément de commutation lorsque le générateur électrochirurgical est inactif et pour ouvrir l'élément de commutation lorsque le générateur électrochirurgical est actif.

7. Générateur électrochirurgical selon l'une quelconque des revendications 4, 5 ou 6, dans lequel le drain de charge comporte une résistance.

8. Générateur électrochirurgical selon l'une quelconque des revendications 4 à 7, dans lequel le drain de charge comporte une thermistance à coefficient de température positif et/ou un fusible réarmable.

9. Générateur électrochirurgical selon la revendication 4, 5 ou 6, dans lequel l'élément de commutation est branché en parallèle avec le condensateur.

10. Générateur électrochirurgical selon la revendication 1, dans lequel un condensateur est placé au niveau d'une prise centrale sur l'enroulement secondaire du transformateur.

11. Générateur électrochirurgical selon l'une quelconque des revendications précédentes, dans lequel le condensateur est intégré dans une carte de circuit imprimé (300).

12. Générateur électrochirurgical selon la revendication 1, comprenant une carte de circuit imprimé (300) comprenant :
une pluralité de contacts (302, 304, 306, 308) recevant l'enroulement secondaire du transformateur, la pluralité de contacts comportant : un premier contact (302) couplé électriquement à une borne active (214) du générateur électrochirurgical ; un deuxième contact (304) couplé électriquement à une borne de retour (216) du générateur électrochirurgical ; un troisième contact (306) ; et un quatrième contact (308) ; et
le condensateur étant intégré dans la carte de circuit imprimé, le condensateur étant couplé électriquement au troisième contact et au quatrième contact.

13. Carte de circuit imprimé selon la revendication 12, dans laquelle le troisième contact est couplé électriquement à la première bobine du transformateur.

14. Carte de circuit imprimé selon la revendication 12 ou 13, dans laquelle le quatrième contact est couplé électriquement à la seconde bobine du transformateur.
